(19) Europäisches Patentamt
European Patent Office
Office européen des brevets

(11) Publication number: 0 420 235 A2

# (12) EUROPEAN PATENT APPLICATION

(21) Application number: 90118563.7

(51) Int. Cl.[5]: C07C 303/22, C07C 309/42

(22) Date of filing: 27.09.90

(30) Priority: 29.09.89 JP 255415/89

(43) Date of publication of application:
03.04.91 Bulletin 91/14

(84) Designated Contracting States:
DE ES FR GB IT

(71) Applicant: KABUSHIKI KAISHA NEOS
2-1, 6-Chome, Kano-Cho, Chuo-Ku
Kobe-Shi, Hyogo-Ken(JP)

(72) Inventor: Aiba, Keizo
Neos Shiga Ryo, Umekage-Cho 3, Kosei-Cho
Koga-Gun, Shiga-Ken(JP)
Inventor: Nakayama, Nobuyuki, Neos
Wakatake Shataku 501
Wakatake-Cho 4, Kosei-Cho
Koga-Gun, Shiga-ken(JP)
Inventor: Shimizu, Hiroaki
Neos Mito Shataku 107, Mito-Cho 6,
Kosei-Cho
Koga-Gun, Shiga-Ken(JP)
Inventor: Tachiiri, Nobuhiko, Neos Wakatake
Shataku 502
Wakatake-Cho 4, Kosei-Cho
Koga-Gun, Shiga-Ken(JP)

(74) Representative: Vossius & Partner
Siebertstrasse 4 P.O. Box 86 07 67
W-8000 München 86(DE)

## (54) A process for preparing aromatic perfluoroalkenyloxy sulfonates.

(57) According to the present invention, there is provided a process for preparing aromatic perfluoroalkenyloxy sulfonates which comprises reacting a perfluoroalkene with a phenolsulfonate or a naphtholsulfonate which may have substituents. The aromatic perfluoroalkenyloxy sulfonates can be prepared through a convenient process in high yield by using the materials which are easily available and easy in handling. Therefore the preparation method is very useful as an industrial process for preparing the aromatic perfluoroalkenyloxy sulfonates.

EP 0 420 235 A2

## A PROCESS FOR PREPARING AROMATIC PERFLUOROALKENYLOXY SULFONATES

The present invention relates to a novel process for preparing aromatic perfluoroalkenyloxy sulfonates.

Although aromatic perfluoroalkenyloxy sulfonates are useful as fluorine-containing surfactant, preparation thereof comes into question from a practical viewpoint.

For example, perfluoroalkenyloxy benzene sulfonates, which have excellent property as fluorine-containing surfactant and are widely used as various kinds of detergents, emulsifiers, additives for resins and the like, are prepared by sulfonating a reaction product of perfluoroalkene and phenol with oleum and neutralizing the sulfonated product ; cf. JP-A- 164199/1982). However, this preparation method has demerits wherein not only yield of said sulfonates is low but also oleum which is dangerous frow a safety point of view and is troublesome in handling must be employed.

It is the object of the present invention to provide a novel process for preparing aromatic perfluoroalkenyloxy sulfonates wherein said sulfonates can be obtained through one process in high yield by using materials which are easy in handling. This object is achieved by the invention.

Therefore, the subject matter of the present invention is a process for preparing aromatic perfluoroalkenyloxy sulfonates which comprises reacting a perfluoroalkene with a phenolsulfonate or naphtholsulfonate which may have substituents.

As the phenolsulfonates employed in the present invention, a compound of the following formula (I) is exemplified:

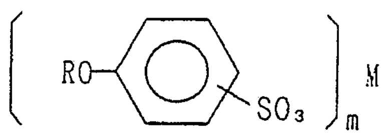

(I)

in which R represents a hydrogen atom or an alkali metal atom, M represents an alkali metal atom, an alkaline-earth metal atom, an ammonium group or pyridinium group and m is an integer of 1 or 2.

In the formula (I), preferred alkali metals are sodium and potassium and preferred alkaline-earth metals are magnesium and calcium. The pyridinium group in the formula (I) may have substituents such as alkyl groups, alkoxy groups of alkenyl groups of one to three carbon atoms and halogeno groups.

The phenolsulfonates of the formula (I) may have other substituents in the aromatic nucleus such as alkyl groups, alkoxy groups, alkenyl groups and halogeno groups.

As the naphtholsulfonates employed in the present invention, a compound of the following formula (II) is exemplified:

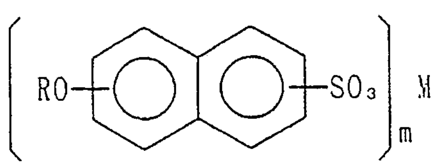

(II)

in which R, M and m have the same meanings as mentioned above.

In the formula (II), preferred alkali metals are sodium and potassium and preferred alkaline-earth metals are magnesium and calcium . The pyridinium group in the formula (II) may have substituents such as alkyl groups, alkoxy groups or alkenyl groups of one to three carbon atoms and halogeno groups.

The naphtholsulfonates of formula (II) may have other substituents in the aromatic nuclei such as alkyl groups, alkoxy groups, alkenyl groups and halogeno groups.

Preferred perfluoroalkenes which react to the aforesaid phenolsulfonates or naphtholsulfonates are compounds of the following formula (III):

$C_nF_{2n}$ (III)

in which n is integer of 6 or 9.

The compounds can be prepared by, for example, dimerization or trimerization of hexafluoropropene. Examples of the dimer or trimer of hexafluoropropene are as follows:

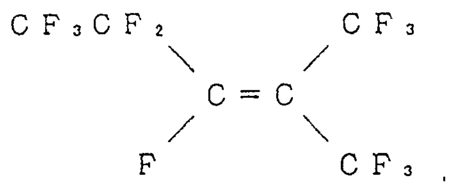

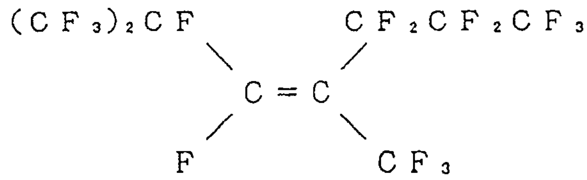

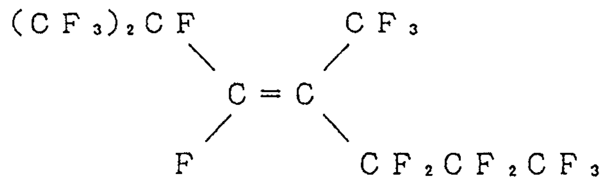

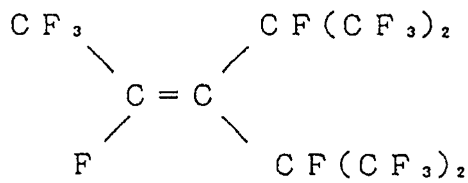

The mole ratio of phenolsulfonate or naphtholsulfonate to perfluoroalkene when reacting the former with the latter is usually 1:1-1:5. The reaction temperature is not restricted and may suitably be selected according to the kind of the reactants. For example, the reaction temperature is -40° C - 0° C, preferably -10° C - 0° C when $C_6F_{12}$ is used, and 10° C - 60° C, preferably 40° C - 60° C when $C_9F_{18}$ is employed.

The above reaction may usually be carried out in an aprotic polar solvent such as N,N-dimethylformamide, N,N-dimethylacetamide and dimethyl sulfoxide.

When the reactant to which the perfluoroalkene reacts has a phenolic hydroxyl group, the above reaction is performed in the presence of a basic catalyst such as a trialkylamine (e.g.triethylamine).

According to the aforesaid reaction, a perfluoroalkenyloxy benzenesulfonates of the formula (IV):

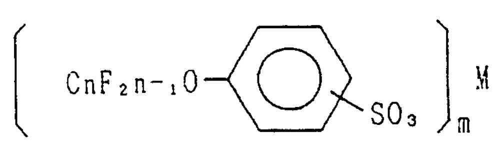 (IV)

in which M, m and n have the aforesaid meanings or a perfluoroalkenyloxy naphthalenesulfonates of the formula (V):

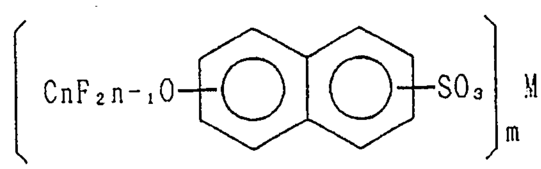 (V)

in which M, m and n have the aforesaid meanings can be obtained.

The above reaction is preferably carried out under substantially unhydrous condition because the yield of the product decreases when water is present in the reaction system. For example, as illustrated in the following examples, the yield of sodium perfluorononenyloxy benzenesulfonate formed by the reaction of dihydrate of sodium p-phenolsulfonate with perfluorononene is lower than that of the product formed by the

reaction of unhydrous sodium p-phenolsulfonate with perfluorononene.

According to the present invention, the aromatic perfluoroalkenyloxy sulfonates can be prepared through one convenient process in high yield by using materials which are easily available and easy in handling. Therefore the preparation method is very useful as an industrial process for preparing the aromatic perfluoroalkenyloxy sulfonates.

The present invention is illustrated by the following examples.

## EXAMPLE

### Example 1

Sodium p-phenolsulfonate (1.96g;10mM) dried at 130° C for 2 hours was dissolved in N,N-dimethylformamide (30ml). Triethylamine (1.01g;10ml) and perfluorononene $C_9F_{18}$ (4.50g;10mM) were added to the solution and the mixture was stirred at 40° C for 2 hours. The reaction mixture was poured into saturated agueous solution of sodium chloride (60ml) to precipitate a solid product, said product being filtered and then dried at 130° C for 2 hours.

According to the titration using methylene blue-chloroform, it was found that 5.34g of sodium perfluorononenyloxy benzenesulfonate are contained in the crude product (yield:85%). The crude product was purified by recrystallization from isopropyl alcohol and / or methyl alcohol.

Spectral data of the product are as follows:

IR(KBr)

1132 - 1306cm$^{-1}$ (C-F)

1192 - 1242cm$^{-1}$ ($SO_3$)

1496, 1594$^{-1}$ cm (benzene ring)

60MHz $^1$H-NMR (in $CD_3OD$ ; hexamethyldisilane as an internal standard)

6.95ppm (2H, m)

7.84ppm (2H, m)

### Example 2

In accordance with the same procedure as described in Example 1 except that dihydrate of sodium p-phenolsulfonate (2.32g;10mM) was used, 4.51g of sodium perfluorononenyloxy benzenesulfonate were obtained (yield:72%).

### Example 3

Disodium p-phenolsulfonate (2.18g;10mM) dried at 130° C for 2 hours was dissolved in N,N-dimethylformamide (30ml). Triethylamine (1,01g;10ml) and perfluorononene $C_9F18$ (4.50g;10mM) were added to the solution and the mixture was stirred at 60° C for 2 hours. The reaction mixture was poured into saturated aqueous solution of sodium chloride (60ml) to precipitate a solid product, said product being filtered and then dried at 130° C for 2 hours.

According to the titration using methylene blue-choloroform, it was found that 5.29g of sodium perfluorononenyloxy benzenesulfonate are contained in the crude product (yield:84%).

### Example 4

Sodium p-phenolsulfonate (1.96g;10mM) dried at 130° C for 2 hours was dissolved in N,N-dimethylformamide (24ml). Triethylamine (1.01g;10mM) and perfluorohexene $C_6F_{12}$ (3.00g;10mM) were added to the solution under agitation, said solution being maintained at -5° C by means of a water bath containing ice and sodium chloride. The mixture was stirred at -5° C for 2 hours and the reaction mixture was post-treated in the same manner as described in Example 1.

According to the titration using methylene blue-chloroform, it was found that 3.57g of sodium perfluorohexenyloxy benzenesulfonate are contained in the crude product (yield:75%). The crude product was purified by recrystallization from isopropyl alcohol and / or methyl alcohol.

Spectral data of the product are as follows:

IR(KBr)
1132 - 1326 $cm^{-1}$ (C-F)
1198 - 1260 $cm^{-1}$ ($SO_3$)
1498, 1596 $cm^{-1}$ (benzene ring)
60MHz $^1$H-NMR (in $CD_3OD$ : hexamethyldisilane as an internal standard)
7.03ppm (2H, m)
7.84ppm (2H, m)

## Claims

1. A process for preparing aromatic perfluoroalkenyloxy sulfonates which comprises reacting a perfluoroalkene with a phenolsulfonate or naphtholsulfonate which may have substituents.

2. A process according to Claim 1, wherein a phenolsulfonate of the formula (I):

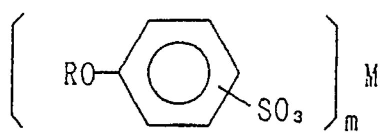

(I)

in which R represents a hydrogen atom or an alkali metal atom, M represents an alkali metal atom, an alkaline-earth metal atom, an ammonium group or pyridinium group and m is an integer of 1 or 2
or a naphtholsulfonate of the formula (II):

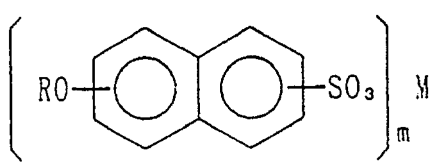

(II)

in which R, M and m have the same meanings as mentioned above
is reacted with a perfluoroalkene of the formula (III):
$C_nF_{2n}$ (III)
in which n is integer of 6 or 9
to form a perfluoroalkenyloxy benzenesulfonate of the formula (IV):

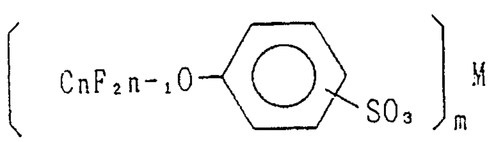

(IV)

in which M,m and n have the aforesaid meanings or a perfluoroalkenyloxy naphthalenesulfonate of the formula (V):

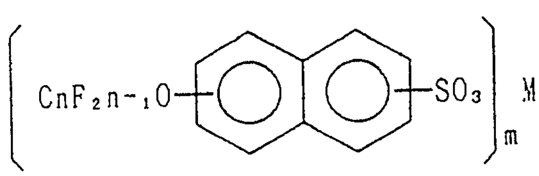

(V)

in which M, m and n have the aforesaid meanings.